# EUROPEAN PATENT SPECIFICATION

(11) **EP 0 945 148 B1**
(45) Date of publication and mention of the grant of the patent: **26.11.2003**
(21) Application number: 98200952.4
(22) Date of filing: 26.03.1998
(51) Int. Cl.: A61L 31/00

(54) **Endovascular stents with a polymeric coating**
Endovaskuläre Gefässprothesen mit polymerer Beschichtung
Prothèse endovasculaire comportant un revêtement polymérique

(43) Date of publication of application: 29.09.1999
(73) Proprietor: Biomat B.V., 6229 ER Maasstricht (NL)
(72) Inventor: Koole, Levinus Hendrik, 6271 EC Gulpen (NL); Bär, Fredericus Wilhelmus Hendricus Maria, 6226 WZ Maastricht (NL); van der Veen, Frederik Hendrik, 3770 Riemst (BE)
(74) Representative: Smulders, Theodorus A.H.J., Ir.

(56) References cited:
- GB-A- 2 190 387
- US-A- 3 861 396
- US-A- 5 674 241

## Description

This invention relates to endovascular stents, and more particularly relates to polymeric coatings, applied to the surface of metallic stents in order to improve their biocompatibility and/or to serve as a depot for the local release of a drug which is intended to inhibit or decrease intimal hyperplasia after intra arterial stent placement.

Percutaneous translumenal angioplasty (PTA) has become a widespread approach for the treatment of arterial disease throughout the human body. The principle is that an atherosclerotic plaque, narrowing the lumen of an artery, is dilated by means of an inflatable balloon. PTA is, however, associated with several disadvantages, due to the fact that atherosclerotic plaques show varying degrees of elasticity. This implies that the lumen may contract again after removal of the balloon; this process is referred to as elastic recoil. Consequently, de success rate of PTA is rather limited. Recurrence of the stenosis occurs with a probability of app. 40 %. These rather disappointing results have stimulated the development of clinically useful endolumenal support devices, usually called *stents*. A stent is a device that is deployed percutaneously to enhance the patency of an artery after PTA. The stent is deployed via either a balloon dilatation (in the case of a balloon-expandable stent), or by releasing the stent structure in a mechanical way (in the case of a self-expanding stent). All stents that are currently in clinical use are metallic objects. Self-expanding stents consist of spring-like metallic wires, and balloon expandable stents are made of plastically deformable alloys. Stents are either wire coils or slotted metallic tubes. Currently, more than 20 different stents are in clinical use. It has been firmly established that the use of stents gives superior acute and long-term results as compared to normal PTA.

A remaining problem with stents is that their surface is thrombogenic; the metallic surface essentially lacks sufficient biocompatibility. Placement of a stent implies the risk of stent-surface-induced thrombus formation. In the longer run, the presence of a metallic surface in the vessel wall may provoke a proliferative reaction of the intimal cell structures near the stent. These reactions are believed to be important risk factors with respect to in-stent restenosis. The risk of thrombosis associated with placement of a stent can be reduced by systemic administration of anticoagulant agents (which, however, increase the bleeding risk), or antiplatelet agents, which inhibit activation and/or aggregation of blood platelets.

The present invention relates to a stent having a polymeric coating which can be used to completely cover the surface thereof, thus leading to a marked improvement of the biocompatibility. The coating is designed such that both significantly reduced overgrowth of the stent with neo-intimal tissue and firm attachment to the stent metal can be achieved.

Application of polymeric coatings on the surface of metallic stents is not unprecedented.

US Patent 5674241 describes a Covered Expanding Mesh Stent in the form of an expandable mesh with an expandable polymeric layer thereon. The resulting device is tubular, not permitting flow or growth of neointimal cells across its wall.

US Patent 5389106 describes a radially expandable stent for intravascular implantation comprising a distensible frame and an impermeable deformable membrane interconnecting portions to the frame to form an impermeable exterior wall.

US Patent 5282823 describes a radially expandable stent for implantation within a body lumen having a generally cylindrical body with open proximal and distal ends, the cylindrical body comprising a plurality of metal elements joined to allow flexing of the cylindrical body along the longitudinal axis of the body whereby the stent can conform to a curved body lumen and a polymeric film extending between the metal elements of the stent. The stent provides a biocompatible polymeric surface to contact and support a body lumen and also a flexible structure to allow the stent to conform closely to bends in a body lumen. The stent is especially useful for repairing an injury to blood vessels caused during angioplasty procedures.

There remains, however, a need for further improvement, particularly with respect to a stent coating which is biocompatible per se, which permits flow and/or growth of neo-intimal cells across its mesh or porous wall structure, which is potentially useful as a depot for drug that are to be released locally after deployment of the stent in the dilated vessel wall.

A family of polymeric biomaterials has been prepared and tested with respect to their potential utility as thin coating material for metallic endovascular stents.

The invention accordingly is directed to a stent as defined in claim 1.

The purpose of this invention was to improve the biocompatibility of the stent surface while preserving the physical/mechanical properties of the stent construction (e.g. self-expanding properties, balloon expandable properties). The polymeric materials are copolymers, terpolymers or tetrapolymersof the poly(acrylate, poly(methacrylate), poly(cyanoacrylate), or a similar type. These materials are prepared from at least two different molecular building blocks (reactive monomers) namely
1. one or more hydrophilic monomers of the (substituted) acrylate type, rendering the coated surface more biocompatible (i.e. less thrombogenic, and less stimulating with respect to neo-intima proliferation), said building block or building blocks having one or more amino or substituted amino groups in their side chain, and
2. one or more hydrophobic monomers of the (substituted) acrylate type, resulting in strong physical adherence of the polymeric coating to the metallic stent, preventing detachment of the coating from the metallic surface.

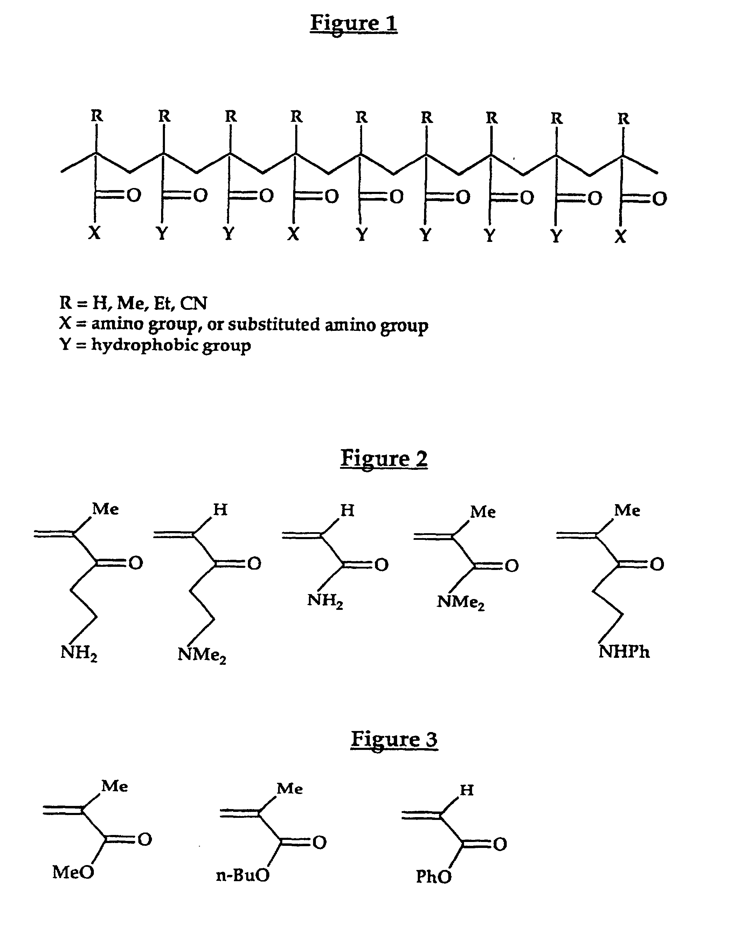

The polymeric materials to be used in this invention are prepared from at least two different monomeric structures of the (substituted) acrylate type. At least one of these monomers has a hydrophilic nature due to the presence of one or more amino groups, alkylated amino groups, arylated amino groups or amino groups that are substituted in another manner, in its side chain. The other type of monomer or monomers have a hydrophobic nature, in its/their side-chain (examples of which are, but are not limited to: alkylacrylates, arylacrylates, alkylmethacrylates, arylmethacrylates). The polymeric materials subject to this invention are represented by the generalised structural formula in the attached Figure 1. in which R = H, Me, Et, CN; X = -NH₂, -CH₂-CH₂-NH₂, -NHMe, -CH₂-CH₂-NHMe, -NMe₂, -CH₂-CH₂-NMe₂, CH₂-NH₂, -CH₂-NHMe, -CH₂-NMe₂, NHEt, CH₂-CH₂-NHEt, NHAr, CH₂-CH₂-NHAr, NAr₂, CH₂-CH₂-NAr₂, or any other group in which one or more amino groups or amino groups carrying one or more organic groups are present;
Y = Me, Et, Pr, i-Pr, Bu, i-Bu, Ph, Ar, or any other hydrophobic organic group.

Specific examples of the monomers to be used in the two groups of materials are given in the following table and the attached figures 2 and 3.

| **Hydrophobic monomer** | **Hydrophilic monomer** |
|---|---|
| Methyl acrylate | Methacryl amide |
| Methyl methacrylate | 2-aminoethyl methacrylate |
| Methyl cyanoacrylate | N-methyl acrylamide |
| Ethyl acrylate | 2-(N-methylamino) ethyl methacrylate |
| Ethyl methacrylate | 2-(N-phenylamino) ethyl methacrylate |
| Ethyl cyanoacrylate | 2-(N-dimethylamino) ethyl methacrylate |
| Phenyl acrylate | 2-(N-diphenylamino) ethyl ethacrylate |
| Phenyl methacrylate | 2-(dimethylamino) ethyl methacrylate |
| Phenyl cyanoacrylate | 2-(N-diphenylamino) ethyl acrylate |
| Butyl acrylate | |
| Butyl methacrylate | |
| Butyl cyanoacrylate | |

The composition of the polymeric materials to be used in this invention, expressed in terms of mole % of X (= 100 - mole % of Y) is in the range 0.1 to 99.9. The relative ratio of the two types of monomers may vary depending on the type of stent, and the level of 2-(N-diphenylamino) ethyl acrylate hydrophylicity and hydrophobicity of the various monomers. Of course it is also possible to use two or more monomers of each group to fine-tune the properties. In addition thereto it is also possible to include subordinate amounts of other monomers, not falling within the scope of the two groups cited above.

Examples of combinations of hydrophobic and hydrophilic monomeric building blocks that lead to particularly effective stent coatings are:
2-(dimethylamino) ethyl methacrylate - methyl methacrylate (preferably in molar ratio 90:10)
methacrylamide - butylmethacrylate (preferably in molar ratio 95:15)
2-(N-diphenylamino) ethyl acrylate - methylcyanoacrylate (preferably in molar ratio 88:12)

The side groups X and Y can be arranged along the main chain of the polymer molecules either in a random fashion, in blocks, or in an alternating fashion. The hydrophilic side groups X are responsible for the biocompatibility of the polymer-coated stent surface, as well as for the (slight) swelling of the polymeric coating occurring upon exposure of the stent to blood. The purpose of the hydrophobic groups Y is to achieve strong adhesion of the coating to the stent structure, such that no parts of the coating separate from the stent structure during or after its deployment. Note that these polymeric materials can also be physically mixed (blended) with other polymeric materials, and that such blends will also show increased suitability in the present invention.

All polymeric materials to be used in this invention can be prepared in a bulk polymerisation process, but it is noted that other polymerisation procedures, (examples of which are, but are not limited to: suspension polymerisation, solution polymerisation or other preparative procedures known in the art) would provide suitable preparations as well.

The copolymers as described above can be dissolved in a volatile organic solvent, and can be applied to the stent via a dip-coating procedure or via a spray process. Other processes resulting in a suitable coating on the metal may also be used.

The metal or metal alloy to be used as basis for the stent depends on the final properties required. Examples of suitable metals and alloys are stainless steel, tantalum, platinum, gold and shape-memory alloys such as nitinol.

In vitro tests as well as animal tests using coated and non-coated (bare) stents revealed the improved biocompatibility of the stent surface, as well as its mechanical stability and integrity. Placement of coated and uncoated self-expanding stents (Wallstent) in the arteria carotis in pigs showed that neo-intimal proliferation is clearly diminished as a consequence of the presence of the coating. This is of great importance with respect to further optimisation of the performance of endovascular stents, by decreasing the risks of complication due to thrombosis and/or in-stent restenosis.

Preparation of the polymeric biomaterials subject to this invention (viz. Figure 1) is outlined in Example 1. Note that his application only refers to a thin coating on the metallic stent surface, i.e. the resulting polymer-coated stents differ principally from the so-called " covered stent" which is known in the art (see, for instance: US Patents 5674241, 5389106, 5282823, *vide supra*). The " covered stent" known in the art has the disadvantage of covering the entire lumen of the blood vessel after its deployment, Consequently, the "covered stent" will block blood flow through small branching arteries at the site of the lesion. The polymer-coated stent that is presently described as a new invention does not have this disadvantage, since it will permit, after its deployment, blood flow through small branching arteries at the site of the lesion to approximately the same extent as the bare (uncoated) stent.

The polymer coating is applied to the surface of the stent in one of the final steps of its manufacturing, but prior to sterilisation. The polymeric biomaterial can be applied as a solution in a volatile organic solvent, via a spray process, a dip-coating process, or otherwise. This may be followed by a treatment of the coated stent at elevated temperature and/or vacuum, in order to facilitate evaporation of residual solvent molecules, and/or in order to achieve firm attachment of the polymer coating to the metallic surface. The application of a primer coating, which is sandwiched between the metallic stent surface and the polymeric biomaterial according to Figure 1, may be advantageous.

### EXAMPLE 1A. Preparation of a polymeric material

The copolymer of 2-(dimethylamino)ethyl methacrylate and methyl methacrylate (mole ratio 90 : 10) was prepared from methyl methacrylate (7.28 g, 72.7 mmol), and 2-(dimethylamino)ethyl methacrylate (102.82 g, 654.3 moles) in a laboratory-scale bulk polymerisation reaction. AIBN (0.044 mole %) was used as the radical initiator.

The reaction mixture was mixed thorougly, and subjected to a heat treatment in a time-temperature controlled reactor under oxygen-free conditions. The temperature profile used was: 60°C (10 h), 85°C (4 h), 100°C (2 h). This afforded the desired polymer as an opaque material. The residual monomer content was measured to be below 0.4 mole % (high-resolution proton NMR analysis). Gel permeation chromatography against polystyrene standards showed: Mw = 81559 Daltons, Mn = 14393 Daltons, Mz = 955259 Daltons; polydispersity = 5.67.

The polymeric material was found to be soluble in volatile organic solvents, such as tetrahydrofuran and methanol.

### EXAMPLE 1B. Preparation of test samples from the material.

Polymeric materials like the one described in Example 1A were used for film casting. Aliquots of the polymer were dissolved in an organic solvent, and the solution was casted into a Petri dish. Evaporation of the solvent at ambient temperature yielded transparent films, which were used for biocompatibility testing. Particularly these foils were used for thrombin generation tests (Journal of Biomedical Materials Research, vol. 29, 917-928 (1995)). Some results are summarized in Table 1.

| Material (compositions mole : mole) | Lag-time for thrombin generation (s) |
|---|---|
| MMA : dimethylaminoethylmethacrylate 1 : 16 | 742 |
| MMA : dimethylaminoethylmethacrylate 1 : 9 | 820 |
| MMA : dimethylaminoethylmethacrylate 1 : 4 | 790 |
| MMA : dimethylaminoethylmethacrylate 1 : 1 | 722 |
| polyurethane | 483 |
| polyvinyl chloride | 660 |

### EXAMPLE 2. Spray coating of the polymers on metallic surfaces.

Dilute solutions of the polymeric materials as described in Example 1A in a volatile organic solvent were used in spray-coating experiments. The solutions were sprayed through a nozzle with pressurized air onto, for example, metallic wires. These were subsequently dried at elevated temperatures. Strong adherence of the polymeric materials to the metallic surfaces was noted. The adherent polymeric coating proved not to dissolve, even during prolongued exposure to hot water (80°C, 24 h - 480 h). This coating procedure was also followed to apply polymeric coatings to the metallic wires of stents, particularly to the self-expanding wall stents. The performance of these polymer-coated stents, versus uncoated (bare) stents was tested in the arteria carotis (in vivo experiments) using a pig model (see Example 3).

### EXAMPLE 3.

The polymer with composition methylmethacrylate / dimethylamino- ethylmethacrylate 1 : 9 was applied as a thin, uniform coating to the struds of a series of metallic self-expanding stents (Wall-stents). An in vivo animal study, aimed at making a comparison of the performance of the coated versus uncoated stents was performed. In these experiments, the arteria carotis in pigs was used as the model system. The study protocol was as follows:
- Study pig is weighed and, if between 10 and 16 kilograms, anaesthetised.
- Femoral access is established, and an angiogram taken. QCA confirms artery size to be between 2.5 and 3.0 mm diameter.
- Midline neck incision exposes both carotid arteries. They are isolated and bathed in lidocaine gel. Initial flow through both arteries is measured five minutes after placement of Doppler probes.
- Probes are removed.
- First carotid artery is crushed. Using a locking forceps, six crushes are administered crosswise, five seconds in duration each with two seconds delay between crushes. The crushes are next to each other along the artery, and the resultant injury is 12-15 mm in length.
- Stent device for first artery is primed for placement.
- Five minutes after the crush injury, the stent is placed across the crush injury.
- Mongoose sized to artery diameter is used to postdilate the stent, using 30 second inflation time.
- Second artery is crushed as above.
- Stent device for second artery is primed for placement.
- Five minutes after injury, the second stent is placed over the injury.
- Mongoose sized to artery diameter is used to post-inflate stent.
- All catheters are removed and Doppler probes replaced on carotids with additional lidocaine jelly. Flow in both carotids, heart rate and blood pressure is followed every second minute for 10 minutes, then every 5 minutes until 1 hour after crush injury administered.
- The probes are removed and the femoral and neck incision closed.
- Carotid flow measured at 1 or 7 days, carotids harvested with one inch proximal and one inch distal vessel surrounding stent. The animal is then sacrificed.

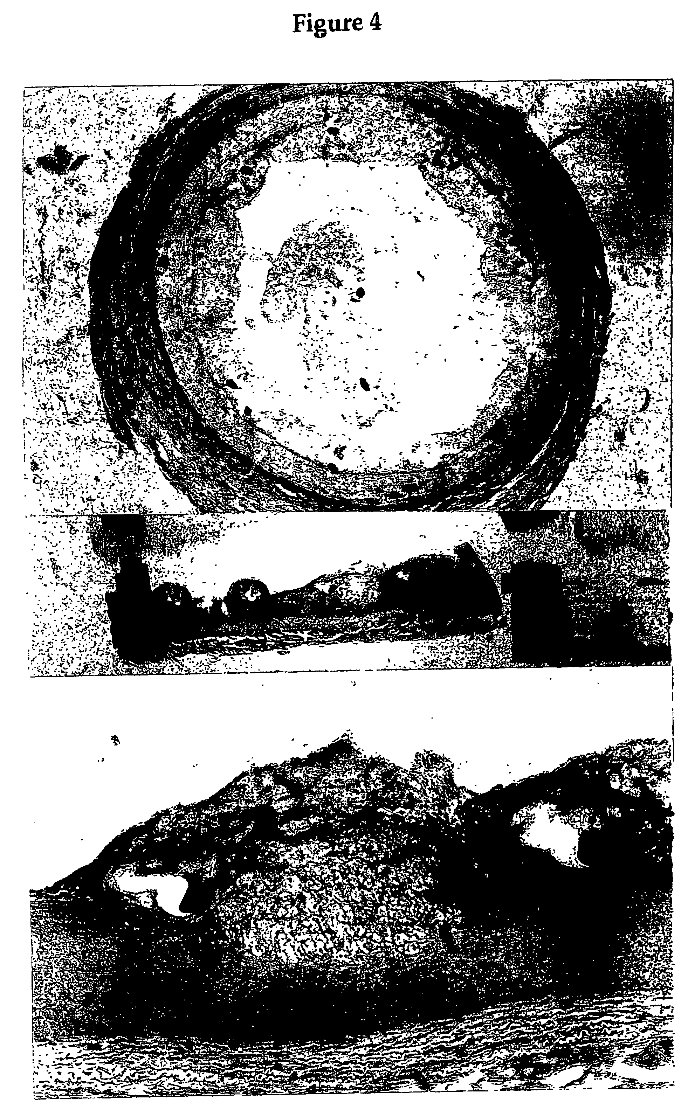

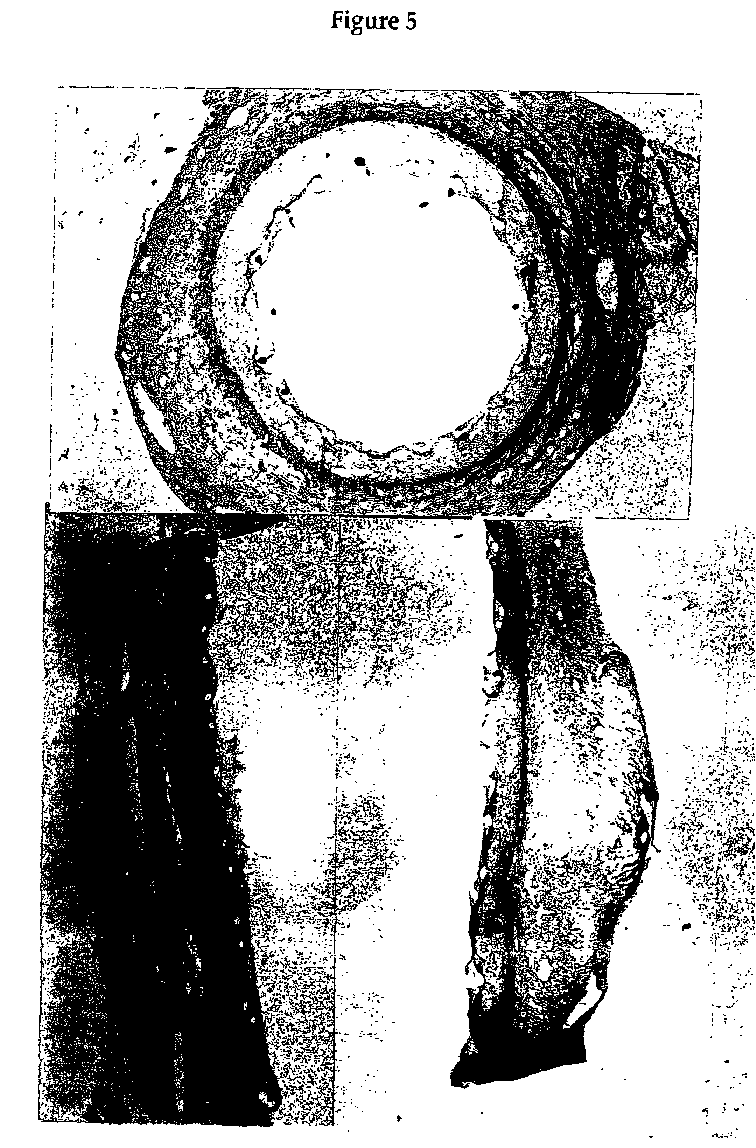

After preparation of microscopic coupes for histological/pathological studies, observations as represented in Figures 4 and 5 were made.

Microscopic coupes for histological/pathological examination were prepared after explantation. These coupes showed clear differences for the coated versus uncoated stents. The coated stents had significantly less formation of neointima and consequently a much wider lumen. For the non-coated (bare) stents occlusion due to thrombus formation was observed in some cases. Figures 4 and 5 show representative microscopic coupes. Figure 4 shows microscopic coupes of a representative implant of a bare (non-coated) stent (arteria carotis, pig model, see text example 3). A pronounced reaction of the vessel wall, provoked by the presence of the stent, is clearly visible.

Figure 5 shows microscopic coupes of a representative implant of a polymer-coated stent (arteria carotis, pig model, see text example 3). Comparison with figure 4 shows that much less neo-intimal tissue is formed, resulting in a substantially larger lumen.

## Claims

1. Endovasculair stent comprising an inner structure, preferably from metal, and a biocompatible thin polymeric coating prepared from at least two different groups of monomers, namely:
one or more hydrophilic monomers of the acrylate type, said monomer or monomers having one or more amino groups in a side chain, and
one or more hydrophobic monomer of the acrylate type.

2. Stent according to claim 1, wherein the molar ratio of the hydrophilic monomers to the hydrophobic monomers ranges from 0.1 - 99.9 to 99.9 to 0.1.

3. Stent according to claim 1 or 2, wherein the hydrophilic monomers are selected from the group of acrylates, methacrylates, ethacrylates, cyanoacrylates, or other acrylate structure having one or more amino or substituted amino groups in their side chain.

4. Stent according to claim 1 or 2, wherein the hydrophobic monomers are selected from the group of acrylates, methacrylates, ethacrylates, cyanoacrylates.

5. Stent according to claims 1-4, wherein the metal is selected from the group of selected from the group consisting of stainless steel, tantalum, gold and nitinol.

6. Stent of claims 1-5, wherein the stent body is expandable via, for instance, a balloon.

7. Stent of claim 6, wherein said expandable stent body has a configuration selected from the group consisting of coiled spring, braided filament, perforated tube, slit tube, and zig-zag.

8. Stent of claims 1-7, wherein said polymeric coating has an additive contained therein.

9. Stent of claim 8, wherein said additive is selected from the group of antibacterial, anti-microbial, anti-calcification, anti-encrustation, growth hormones, anti-cancer-related drugs, medicaments, anti-thrombogenic agents, and anti-restenosis agents.

10. Stent of claims 1-10, wherein said polymeric coating inhibits tissue ingrowth through walls of the stent.

## Patentansprüche

1. Endovaskulärer Stent, umfassend eine innere Struktur, vorzugsweise aus Metall, und eine biokompatible dünne polymere Beschichtung, die aus zumindest zwei verschiedenen Gruppen von Monomeren hergestellt ist, nämlich:
einem oder mehreren hydrophilen Monomeren vom Acrylat-Typ, wobei das Monomer oder die Monomere eine oder mehrere Amino-Gruppen in einer Seitenkette aufweisen, und
einem oder mehreren hydrophoben Monomeren vom Acrylat-Typ.

2. Stent gemäß Anspruch 1, wobei das molare Verhältnis der hydrophilen Monomere und der hydrophoben Monomere von 0.1-99.9 bis 99.9 zu 0.1 reicht

3. Stent gemäß Anspruch 1 oder 2, wobei die hydrophilen Monomere ausgewählt sind aus der Gruppe der Acrylate, Methacrylate, Ethacrylate, Cyanoacrylate oder anderer Acrylat-Struktur mit einer oder mehreren Amino- oder substituierten Amino-Gruppen in ihrer Seitenkette.

4. Stent gemäß Anspruch 1 oder 2, wobei die hydrophoben Monomere ausgewählt sind aus der Gruppe der Acrylate, Methacrylate, Ethacrylate, Cyanoacrylate.

5. Stent gemäß den Ansprüchen 1 bis 4, wobei das Metall ausgewählt ist aus der Gruppe, die aus rostfreiem Stahl, Tantal, Gold und Nitinol besteht.

6. Stent nach Ansprüchen 1 bis 5, wobei der Stentkörper beispielsweise über einen Ballon erweiterbar ist.

7. Stent nach Anspruch 6, wobei der erweiterbare Stentkörper eine Konfiguration aufweist, die ausgewählt ist aus der Gruppe, die aus Wendelfeder, geflochtenem Filament, durchlöchertem Rohr, Schlitzrohr und Zick-Zack besteht.

8. Stent nach Ansprüchen 1-7, wobei die polymere Beschichtung ein Additiv enthält.

9. Stent nach Anspruch 8, wobei das Additiv ausgewählt ist aus der Gruppe der antibakteriellen, antimikrobialen, Anti-Kalzifizierungs-. Antiverkrustungs-, Wachstumshormonem, Anti-Krebs-bezogenen Drogen, Medikamente, Antithrombosemittel und Anti-Restenose-Agentien.

10. Stent nach Ansprüchen 1-10. wobei die polymere Beschichtung ein Gewebeeinwachsen durch Wände des Stents hemmt.

## Revendications

1. Prothèse endovasculaire comprenant une structure interne, de préférence en métal, et un revêtement polymère mince biocompatible préparé à partir de deux groupes différents de monomères, c'est-à-dire :
un ou plusieurs monomères hydrophiles du type acrylate, ledit monomère ou lesdits monomères ayant un ou plusieurs groupes amino dans une chaîne latérale, et
un ou plusieurs monomères hydrophobes du type acrylate.

2. Prothèse selon la revendication 1, dans laquelle le rapport molaire des monomères hydrophiles aux monomères hydrophobes varie de 0,1 - 99,9 à 99,9 à 0,1.

3. Prothèse selon la revendication 1 ou 2, dans laquelle les monomères hydrophiles sont choisis à partir du groupe d'acrylates, méthacrylates, éthacrylates, cyanoacrylates, ou d'autre structure acrylate ayant un ou plusieurs groupes amino ou amino substitués dans leur chaîne latérale.

4. Prothèse selon la revendication 1 ou 2, dans laquelle les monomères hydrophobes sont choisis à partir du groupe d'acrylates, méthacrylates, éthacrylates, cyanoacrylates.

5. Prothèse selon les revendications 1 - 4, dans laquelle le métal est choisi à partir du groupe choisi à partir du groupe constitué d'acier inoxydable, de tantale, d'or et de nitinol.

6. Prothèse des revendications 1 - 5, dans laquelle le corps de prothèse peut subir une expansion via, par exemple, un ballon.

7. Prothèse de la revendication 6, dans laquelle ledit corps de prothèse expansible a une configuration choisie à partir du groupe constitué de ressort en spirale, filament tressé, tube perforé, tube fendu et zig-zag.

8. Prothèse des revendications 1 - 7, dans laquelle ledit revêtement polymère a un additif contenu dans celui-ci.

9. Prothèse de la revendication 8, dans laquelle ledit additif est choisi à partir du groupe d'antibactérien, antimicrobien, anticalcification, anti-incrustation, hormones de croissance, médicaments apparentés anticancéreux, médicaments, agents antitrombogènes et agents anti-resténoses.

10. Prothèse des revendications 1 - 10, dans laquelle ledit revêtement polymère inhibe les tissus en croissance par l'intermédiaire des parois de la prothèse.
